# EUROPÄISCHE PATENTSCHRIFT

(11) **EP 2 751 428 B1**
(45) Veröffentlichungstag und Bekanntmachung des Hinweises auf die Patenterteilung: **13.01.2016**
(21) Anmeldenummer: 12772335.1
(22) Anmeldetag: 16.10.2012
(51) Int. Cl.: F04B 43/00, F04B 43/08, F04B 43/12, F04B 45/06, F04B 45/08, A61M 39/10, F16L 33/00, F04B 43/06

(54) **PUMPENSCHLAUCH FÜR EINE PERISTALTISCHE PUMPE**
PUMP HOSE FOR A PERISTALTIC PUMP
TUYAU FLEXIBLE DE POMPAGE POUR UNE POMPE PÉRISTALTIQUE

(30) Priorität: 11.11.2011 EP 11188816
(43) Veröffentlichungstag der Anmeldung: 09.07.2014
(73) Patentinhaber: Connectors Verbindungstechnik AG, 8317 Tagelswangen (CH)
(72) Erfinder: DI LEO, Vito, CH-8302 Kloten (CH)
(74) Vertreter: Schmauder & Partner AG Patent- & Markenanwälte VSP
(86) Internationale Anmeldenummer: PCT/EP2012/070466
(87) Internationale Veröffentlichungsnummer: WO 2013/068206

(56) Entgegenhaltungen:
- EP-A1- 0 388 596
- EP-A1- 0 526 920
- DE-A1- 10 131 563
- DE-A1-102004 024 641
- DE-U1- 9 208 212

## Beschreibung

### Technisches Gebiet

Die Erfindung betrifft einen Pumpenschlauch, insbesondere einen Silikonpumpenschlauch, für eine peristaltische Pumpe.

### Stand der Technik

Peristaltische Pumpen dienen dazu, durch einen Quetschvorgang durch einen Pumpenschlauch eine Vorwärtsbewegung einer Flüssigkeit zu bewirken. Solche peristaltische Pumpen umfassen ein Pumpensegment, in das der Schlauch üblicherweise eingelegt wird. Das Pumpensegment ist dann mit einer Antriebseinrichtung für den peristaltischen Quetschvorgang versehen. Der pumpenschlauch weist üblicherweise Anschlusseinrichtungen auf, die an die Elemente zum Versorgen der Pumpe mit der Flüssigkeit und dem Abnehmer versehen sind. Der Vorteil einer solchen peristaltischen Pumpe ist es, dass wesentliche Teile des Pumpensegmentes, insbesondere das Antriebssegment, nicht mit der peristaltisch zu pumpenden Flüssigkeit in Verbindung kommen. Dies ist besonders vorteilhaft auf dem Gebiet der Biotechnologie, bei der die zu pumpende Flüssigkeit steril sein soll. Aber auch bei kontaminierten Flüssigkeiten auf dem Gebiet der Biotechnologie oder auch in der Technik radioaktiv kontaminierter Flüssigkeiten ist eine solche peristaltische Pumpe vorteilhaft.

Der Pumpenschlauch ist dabei üblicherweise als Wegwerfteil ausgebildet, der normalerweise steril angeliefert wird und nach einer allfälligen Kontamination nicht weiterverwendet werden muss.

Aus der US 5 213 483 A ist eine peristaltische Pumpe mit einem Pumpenkopfgehäuse der genannten Art bekannt. Aus der US 5 388 972 A ist ein Präzisionspumpenkopf bekannt.

Aus der EP 0 388 596 A1, welches als nächstkommender Stand der Technik angesehen wird, ist ein Silikonpumpenschlauch für eine peristaltische Pumpe bekannt, zum Einlegen in ein peristaltisches Pumpensegment mit einem schlauchförmigen Mittelteil und beidseitig angeordneten Anschlussteilen zum Einlegen bzw. Anschliessen des Pumpenschlauches. Die dichtenden Stirnflächen ragen etwas in den Schlauch hinein. An einer der Stirnseiten ist der Schlauch im Bereich seiner Anschlussteile von einer in etwa kreisförmigen Dichtwulst umgeben. Aus der DE 101 31 563 A1 ist ein Schlauch mit beidseitig identischen Anschlussteilen bekannt.

In der EP 1 0 48 848 A1 wird ein peristaltischer Pumpenkopf vorgeschlagen, bei dem der Flansch fluiddicht in einer Ausnehmung eingepasst ist. Dies hat sich aber nicht in jedem Fall als vorteilhaft herausgestellt, da dabei bestimmte Belastungen der peristaltischen Pumpe auftreten können, die vermieden werden können.

Andererseits hat sich die Ausführung der EP 1 048 848 A1 als sehr nachteilig herausgestellt. Dort wird nämlich vorgeschlagen, dass ein Endfitting, also ein Anschlussteil auf den Pumpenschlauch aufgespritzt oder an diesen angebracht wird. Damit sind aber - innerhalb des Schlauches - Übergänge nicht zu vermeiden. Diese sollen aber - als Teil der Aufgabe der Erfindung - zumindest vermeidbar sein sollen.

### Darstellung der Erfindung

Eine Aufgabe der vorliegenden Erfindung ist es, einen gegenüber dem Stand der Technik verbesserten Pumpenschlauch vorzuschlagen, der für eine peristaltische Pumpe geeignet ist und durch den Übergänge im Inneren des Schlauches vermeidbar sind. Die Aufgabe der Erfindung wird durch den Pumpenschlauch nach Anspruch 1 gelöst. Dabei haben die Massnahmen der Erfindung zunächst einmal zur Folge, dass kein Aufspritzen oder Anbringen eines Anschlussteils (Fitting) notwendig ist und die oben beschriebenen Probleme gelöst sind. Die Ausgestaltung des Pumpenschlauches aus Silikon, vorzugsweise aus platinvernetztem Silikon, führt zu einer Erhöhung der Qualität. Dadurch, dass die Anschlussteile jeweils kopfartig eine Dichtfläche aufweisen und die Dichtfläche mit einer im Wesentlichen kreisförmigen Dichtwulst umgeben ist, die longitudinal über die Dichtfläche herausragt, ist ein Anbringen der Anschlussteile an die Elemente zum Versorgen der Pumpe mit der Flüssigkeit und dem Abnehmer direkt und besonders vorteilhaft möglich. Zur Vermeidung von Druckschwankungen etc. ist es vorteilhaft, wenn die Flanschteile Mittel zum nicht gasdichten, vorzugsweise auch zum nicht fluiddichten Einlegen des Pumpenschlauches in das peristaltische Pumpensegment aufweisen. Solche Mittel können zum schlauchförmigen Mittelteil hin ausgebildete Noppen umfassen, die einen kleinen Schlupf sicherstellen.

Vorteilhafte Ausgestaltungen des erfindungsgemässen Gegenstandes sind in den abhängigen Ansprüchen 2 bis 5 angegeben.

Vorteilhaft ist es, wenn das Innere des Pumpenschlauches übergangsfrei zwischen den beiden Dichtflächen ausgebildet ist. Selbstverständlich ist dies nicht notwendig. Möglich wäre es auch, bestimmte Übergänge vorzusehen, wenn dies ausnahmsweise gewünscht wird.

Um ein erleichtertes Anflanschen zu ermöglichen, ist es vorteilhaft, wenn die die Flanschteile (17, 18) von der Dichtwulst (20) einen Abstand aufweisen, beispielsweise von ca. 35mm.

Zur Erleichterung des Anschlusses die an die Elemente zum Versorgen der Pumpe mit der Flüssigkeit und dem Abnehmer wird der Pumpenschlauch vorteilhafter Weise mit einem die Dichtwulst zumindest teilweise überragenden Haltering, vorzugsweise aus einem Hartkunststoff versehen. Dieser Haltering aus hartem Material kann dann axial nach vorne bzw. hinten die Dichtwulst und damit die Dichtfläche an die entsprechende Gegenfläche der Elemente zum Versorgen der Pumpe mit der Flüssigkeit und des Abnehmers drücken, ohne dass eine direkte Krafteinwirkung auf den weichen Silikonschlauch ausgeübt werden müsste.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Figuren

Ausführungsbeispiele der Erfindung werden nachfolgend anhand von Zeichnungen näher beschrieben, dabei zeigen:
- Figur 1: eine Seitenansicht eines erfinderischen Pumpenschlauches,
- Figur 2: eine Detailansicht eines der Flanschteile mit dem Anschlussteil gemäss Figur 1, von der Seite;
- Figur 3: eine Detailansicht von Figur 2 mit dem Flanschteil mit den Noppen, von der Seite; und
- Figur 4: eine Sicht auf den Flanschteil des Pumpenschlauches vom Mittelteil aus gesehen, mit den Abstandsnoppen.

### Wege zur Ausführung der Erfindung

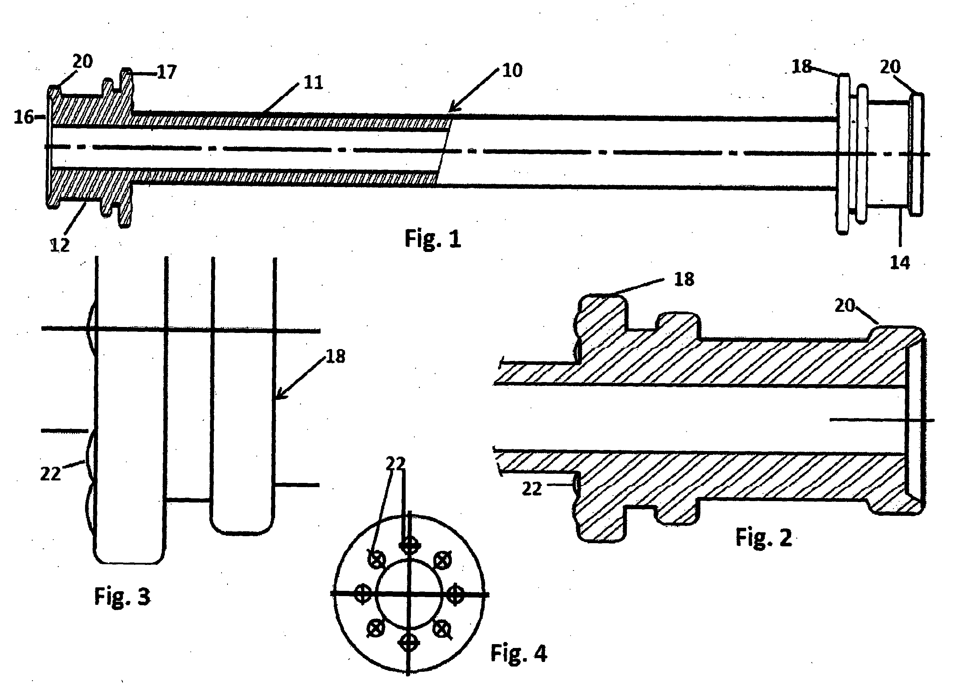

Gemäss einem bevorzugten Ausführungsbeispiel, wie es in Figur 1 dargestellt ist, zeigt einen Pumpenschlauch 10, nämlich einen Silikonpumpenschlauch für eine peristaltische Pumpe. Der Pumpenschlauch 10 besteht grundsätzlich aus fünf Teilbereichen, nämlich
einen schlauchförmigen Mittelteil 11, beidseitig angeordnete Anschlussteile 12 und 14, zum Anschluss des Pumpenschlauches 10 an weitere Elemente, nämlich an die Elemente zum Versorgen der Pumpe mit der Flüssigkeit und dem Abnehmer der gepumpten Flüssigkeit, die nicht dargestellt sind und beidseitig angeordnete Flanschteile 17 und 18 zum Einlegen in einen Kassettenteil der peristaltischen Pumpe.

Alle fünf Teilbereiche des Silikonpumpenschlauch, nämlich der schlauchförmige Mittelteil 11, die beidseitig angeordneten Anschlussteile 12 und 14 und die beidseitig zwischen dem Mittelteil und den Anschlussteilen angeordneten Flanschteilen 17 und 18 sind einstückig aus Silikon, im vorliegenden Ausführungsbeispiel aus platinvernetztem Silikon hergestellt. Dadurch ist der Silikonpumpenschlauch besonders gut sterilisierbar, insbesondere durch Flammensterilisation, ohne dass z.B. aufgespritzte oder anders angebrachte Anschlussteile (Fittings) sich lösen oder sonst wie beschädigt werden könnten. Ausserdem können durch die einstückige Herstellung Übergänge vermieden werden. Durch die einstückige Herstellungsweise ist es zudem möglich, wie im vorliegenden Ausführungsbeispiel die Anschlussteile 12 und 14 jeweils kopfartig jeweils mit einer Dichtfläche 16 zu versehen. Da die Dichtflächen 16 Teil des einstückigen Schlauches sind, kann auf Dichtringe, die sich bei den Schläuchen gemäss dem Stand der Technik als kontaminationsträchtig - vor und nach der Benutzung - verzichtet werden. Im Ausführungsbeispiel sind die Dichtflächen 16 mit einer im Wesentlichen kreisförmigen Dichtwulst 20 umgeben, die longitudinal über die Dichtfläche 16 herausragt.

Im Ausführungsbeispiel ist die Dichtwulst 20 ist gegenüber der Dichtfläche, also nach innen hin, um ca. 70° angefast.

Die Flanschteile 17 und 18 sind im Ausführungsbeispiel ca. 35 mm von der Dichtfläche entfernt, gemessen an der der Dichtfläche abgewandten Flächen. Diese, der Dichtfläche abgewandten Flächen der Flanschteile 17 und 18 weisen im vorliegenden Ausführungsbeispiel Mittel auf zum nicht gasdichten und auch nicht fluiddichten Einlegen des Pumpenschlauches 10 in das peristaltische Pumpensegment. Damit werden für die durch die Elastizität des Materials, also des Silikons und der damit flexiblen Längen entstehenden Bewegungen, insbesondere bei den peristaltischen Bewegungen, möglicherweise auftretenden Verspannungen vermieden oder vermindert. Im vorliegenden Ausführungsbeispiel sind diese Mittel durch Noppen 22, nämlich acht im Umfang verteilte Noppen mit eine Erhebung von ca. 0.5 bis 1 mm ausgebildet.

Im vorliegenden Ausführungsbeispiel ist ein die Dichtwulst 20 teilweise überragenden Haltering (nicht dargestellt) aus einem Hartkunststoff zum Befestigen des Pumpenschlauches 10 an die weitere Elemente der peristaltischen Pumpe vorgesehen. Der Haltering ist verschiebbar zwischen dem Flanschteil 17 bzw. 18 und der jeweiligen Dichtwulst 20 angeordnet. Durch diesen Haltering können beim Anschliessen auftretende, das Silikonmaterial möglicherweise schädigende Kräfte vermieden werden, da diese vom Haltering aufgenommen werden.

### Bezugszeichenliste

- 10: Pumpenschlauch
- 11: Mittelteil
- 12: Anschlussteil
- 14: Anschlussteil
- 16: Dichtfläche
- 17: Flanschteil
- 18: Flanschteil
- 20: Dichtwulst
- 21: das Innere des Pumpenschlauches
- 22: Noppen

## Patentansprüche

1. Pumpenschlauch (10), insbesondere Silikonpumpenschlauch, für eine peristaltische Pumpe, zum Einlegen in ein peristaltisches Pumpensegment mit
- einem schlauchförmigen Mittelteil (11),
- beidseitig angeordneten Anschlussteilen (12, 14), zum Anschluss des Pumpenschlauches (10) an weitere Elemente,
- beidseitig angeordneten Flanschteilen (17, 18), insbesondere zum Einlegen in einen Kassettenteil der peristaltischen Pumpe, wobei
- der schlauchförmige Mittelteil (11), die beidseitig angeordneten Anschlussteile (12, 14) und die beidseitig angeordneten Flanschteile (17, 18) einstückig aus Silikon, vorzugsweise aus platinvernetztem Silikon, hergestellt sind,
- die Anschlussteile (12, 14) jeweils kopfartig eine Dichtfläche (16) aufweisen,
- wobei die Dichtfläche (16) mit einer im Wesentlichen kreisförmigen Dichtwulst (20) umgeben ist, die longitudinal über die Dichtfläche (16) herausragt, **dadurch gekennzeichnet, dass**
die Flanschteile (17, 18) Mittel zum nicht gasdichten, vorzugsweise auch zum nicht fluiddichten Einlegen des Pumpenschlauches (10) in das peristaltische Pumpensegment aufweisen und die genannten Mittel zum schlauchförmigen Mittelteil (11) hin ausgebildete Noppen (22) umfassen.

2. Pumpenschlauch nach Anspruch 1, **dadurch gekennzeichnet, dass** das Innere (21) des Pumpenschlauches übergangsfrei zwischen den beiden Dichtflächen (16) ausgebildet ist.

3. Pumpenschlauch nach Anspruch 1 oder 2, **dadurch gekennzeichnet, dass** beim Anschlussteil (14) die Flanschteile (17, 18) von der Dichtfläche (16) einen Abstand aufweisen.

4. Pumpenschlauch nach Anspruch 3, **dadurch gekennzeichnet, dass** der Abstand ca. 35mm beträgt.

5. Pumpenschlauch nach einem der Ansprüche 1 bis 3, **gekennzeichnet durch** einen die Dichtwulst (20) zumindest teilweise überragenden Haltering, vorzugsweise aus einem Hartkunststoff, zum Befestigen des Pumpenschlauches (10) an die weitere Elemente der peristaltischen Pumpe, dass das Innere (21) des Pumpenschlauches übergangsfrei zwischen den beiden Dichtflächen (16) ausgebildet ist, wobei der Haltering verschiebbar zwischen dem Flanschteil (17, 18) und der Dichtwulst angeordnet ist.

## Claims

1. A pump hose (10), particularly a silicone pump hose, for a peristaltic pump, for insertion into a peristaltic pump segment, comprising
- a tubular center part (11),
- connecting parts (12, 14) arranged at both sides thereof, for connecting the pump hose (10) to other elements,
- flange parts (17, 18) arranged at both sides thereof, particularly for insertion into a cartridge part of the peristaltic pump,
wherein
- the tubular center part (11), the connecting parts (12, 14) arranged at both sides thereof and the flange parts (17, 18) arranged at both sides thereof are integrally produced from silicone, preferably from platinum-linked silicone,
- the connecting parts (12, 14) each have a head-like shaped sealing surface (16),
- wherein the sealing surface (16) is surrounded by a substantially circular sealing bulge (20) that protrudes longitudinally beyond the sealing surface (16), **characterized in that**
- the flange parts (17, 18) comprise means for inserting the pump hose (10) in a non-gas-tight manner, preferably also in a non-fluid-tight manner, into the peristaltic pump segment, and the said means comprise nubs (22) that are formed towards the tubular center part (11).

2. Pump hose according to claim 1, **characterized in that** the inside (21) of the pump hose is configured transitionless between the two sealing surfaces (16).

3. Pump hose according to claim 1 or 2, **characterized in that** in the connecting part (14) the flange parts (17, 18) have a distance from the sealing surface (16).

4. Pump hose according to claim 3, **characterized in that** the distance is about 35 mm.

5. Pump hose according to one of claims 1 to 3, **characterized by** a retaining ring that at least partially projects over the sealing bulge (20) and is preferably made of a hard plastic, for attaching the pump hose (10) to the other elements of the peristaltic pump, the inside (21) of the pump hose being configured transitionless between the two sealing surfaces (16), the retaining ring being slidingly arranged between the flange part (17, 18) and the sealing bulge.

## Revendications

1. Tuyau de pompe (10) en particulier tuyau de pompe en silicone destiné à une pompe péristaltique, pour permettre son insertion dans un segment de pompe péristaltique comprenant :
- une partie médiane (11) en forme de tuyau,
- des parties de connexion (12, 14) situées de part et d'autre de cette partie médiane pour permettre de connecter le tuyau de pompe (10) à d'autres éléments,
- des parties de bride (17, 18) situées de part et d'autre de la partie médiane en particulier pour permettre l'insertion dans une partie de caisson de la pompe péristaltique, tubulure de pompe dans laquelle :
la partie médiane en forme de tuyau (11), les parties de connexion (12, 14) situées de part et d'autre et les parties de bride (17, 18) situées de part et d'autre sont réalisées en une seule pièce en silicone, de préférence en silicone réticulé par du platine,
- les parties de connexion (12, 14) comportent respectivement côté tête une surface d'étanchéité (16),
- la surface d'extrémité (16) est entourée d'un bourrelet d'étanchéité (20) essentiellement circulaire qui fait saillie longitudinalement sur les surfaces d'extrémité (16),
**caractérisé en ce que**
les parties de bride (17, 18) comportent des moyens permettant l'insertion du tuyau de pompe (10) de façon non étanche aux gaz et de préférence également non étanche aux fluides dans le segment de pompe péristaltique, et ces moyens renferment des bossages (22) s'étendant vers la partie médiane en forme de tuyau (11).

2. Tuyau de pompe conforme à la revendication 1,
**caractérisé en ce que**
la partie interne (21) du tuyau de pompe est réalisée sans transition entre les deux surfaces d'étanchéité (16).

3. Tuyau de pompe conforme à la revendication 1 ou 2,
**caractérisé en ce que**
dans les parties de connexion (14) les parties de bride (17, 18) sont situées à distances de la surface d'étanchéité (16).

4. Tuyau de pompe conforme à la revendication 3,
**caractérisé en ce que**
la distance est de l'ordre de 35 mm.

5. Tuyau de pompe conforme à l'une des revendications 1 à 3,
**caractérisé par**
une bague de retenue de préférence en une matière plastique dure dépassant au moins partiellement du bourrelet d'étanchéité (20) pour permettre de fixer le tuyau de pompe (10) aux autres éléments de la pompe péristaltique, la partie interne (21) du tuyau de pompe étant réalisée sans transition entre les deux surfaces d'étanchéité (16) et la bague de retenue autant montée coulissante entre la partie de bride (17, 18) et le bourrelet d'étanchéité.
